# EUROPEAN PATENT SPECIFICATION

(11) **EP 2 382 340 B1**
(45) Date of publication and mention of the grant of the patent: **20.03.2013**
(21) Application number: 10700390.7
(22) Date of filing: 15.01.2010
(51) Int. Cl.: A61L 31/14, A61L 31/02, C25F 3/16

(54) **METHOD AND SOLUTION FOR ELECTROPOLISHING STENTS MADE OF HIGH STRENGTH MEDICAL ALLOYS**
VERFAHREN UND LÖSUNG ZUM ELEKTROPOLIEREN VON STENTS AUS HOCHFESTEN MEDIZINISCHEN LEGIERUNGEN
PROCÉDÉ ET SOLUTION POUR LE POLISSAGE ÉLECTROLYTIQUE DE STENTS FAITS D'ALLIAGES MÉDICAUX À HAUTE RÉSISTANCE

(30) Priority: 16.01.2009 US 145460 P
(43) Date of publication of application: 02.11.2011
(73) Proprietor: Abbott Laboratories Vascular Enterprises Limited, Dublin 2 (IE)
(72) Inventor: WULF, Elena, 72393 Burladingen (DE)
(74) Representative: Peters, Hajo
(86) International application number: PCT/EP2010/000217
(87) International publication number: WO 2010/081724

(56) References cited:
- WO-A1-01/71068
- US-A- 3 190 822
- US-A1- 2005 209 117
- US-A1- 2007 209 947

## Description

### BACKGROUND OF THE DISCLOSURE

The present disclosure relates generally to providing an apparatus and method for electropolishing products made from metals, and in particular, electropolishing metallic medical devices such as stents, made of high strength medical alloys like alloys including e.g. titanium, tungsten, tantalum, niobium, cobalt-chromium, etc. While the apparatus and method are described herein as being applicable mainly to medical stents, in particular intravascular stents, the disclosure is not limited to such medical products. For example, the methods may be applied to electropolish metallic automotive or aerospace components.

Stents are generally tube-shaped devices placed within a blood vessel or other body lumen to maintain the patency of the lumen and, in some cases, to reduce the development of restenosis. The stents may be formed in a variety of configurations which are typically expandable since they are delivered in a compressed form to the desired site. Such a configuration may be a helically wound wire, wire mesh, weaved wire, serpentine stent, chain of rings, other configuration, or combinations thereof. The walls of stents are typically perforated in a framework design of wire-like connected elements or struts or in a weave design of cross-threaded wire. Some stents are made of more than one material. The stent may be, for example, a sandwich of metals having outer layers of a biocompatible material, such as cobalt chromium, with an inner layer providing the radiopacity to the stent for tracking by imaging devices during placement. A stent made of such material may be, for example, a thin layer of titanium between layers of cobalt chromium. In forming such stents from metal, a roughened outer surface of the stent may result from the manufacturing process. It may be desirable for the surface of the stent to be smooth so that it may be more inserted and traversed with reduced friction through the blood vessels or other body lumens toward the site of implantation. A rough outer surface may not only increase frictional obstruction, but may also damage the lining of the vessel wall during insertion. Furthermore, smooth surfaces may reduce thrombus formation and/or corrosion.

Since processing to form metallic stents often results in a product initially having burrs, sharp ends, debris, slag material from melting the metal during processing, other features, or combinations thereof, as a first order treatment of the product, the surface may be descaled in preparation for, for example, further surface treatment such as electropolishing.

An apparatus and method is provided for electropolishing stents after they have been descaled, for example, by the method disclosed in US-A-200709947.

Descaling may include, for example, dipping the stent into a strongly acidic solution and/or ultrasonically cleaning the stent.

Electropolishing is an electrochemical process by which some of the surface metal may be electrolytically dissolved. In general, the metal stent serves as an anode and is connected to a power supply while immersed in an electrolytic solution having a metal cathode connected to the negative terminal of the power supply. Current therefore flows from the stent, as the anode, causing it to become polarized. The rate at which the metal ions on the stent are dissolved may be controlled by the applied current and/or voltage. The positioning of the cathode relative to the stent may provide an even distribution of current to the stent. According to the theory of electropolishing, the current density is typically highest at high points protruding from a surface and is typically lowest at the surface low points. Thus, the higher current density at the raised points may cause the metal to dissolve faster at these points, which may level the surface. Electropolishing therefore may smooth the surface, even to the point where it is shiny and reflective.

The present disclosure provides an apparatus and process for electropolishing a plurality of metallic devices such as stents simultaneously to consistently produce smooth surfaces.

### BRIEF SUMMARY

An embodiment of an apparatus is provided for simultaneously electropolishing a plurality of metallic stents. The apparatus may include a plurality of elongated members having a longitudinal axis, each of the members may include an electrically conductive adaptor capable of being removably affixed to and in electrical contact with a metallic stent; an electrolytic solution; a continuous cathode configured to be located in close proximity to each of the elongated members when the elongated members and cathode are immersed in the electrolytic solution; a cathode current conducting member attached to said cathode; and an anode current conducting member wherein each of the elongated members is conductively connected electrically with the anode current conducting member.

In one embodiment, the cathode has a spiral configuration and/or the electrolytic solution is contained in a container made from non-metallic material. The container may be made from glass, ceramics, plastic, or other materials.

In another embodiment, the apparatus includes a second continuous cathode configured to be located in close proximity to each of the elongated members when the elongated members and cathode are immersed in the electrolytic solution and a second cathode current conducting member attached to the second cathode.

In a further embodiment, the two cathodes are both in the shape of a spiral and are disposed substantially concentrically in the solution.

An embodiment of a method is also provided for simultaneously electropolishing a plurality of metallic stents. The method may include:
a) affixing a stent on each of one or more electrically conductive adaptors of an apparatus, the apparatus including:
   a plurality of elongated members having a longitudinal axis, each of the members including an electrically conductive adaptor capable of being removably affixed to and in electrical contact with a metallic stent;
   an electrolytic solution;
   a continuous cathode configured to be located in close proximity to each of the elongated members when the elongated members and cathode are immersed in the electrolytic solution;
   a cathode current conducting member attached to the cathode;
   an anode current conducting member wherein each of the elongated members is conductively connected electrically with the anode current conducting member;
b) immersing said stents into the electrolytic solution;
c) supplying a voltage difference between the cathode current conducting member and the anode current conducting member;
d) removing the stents from the solution and rinsing with at least one rinsing solution;
e) optionally, repeating steps b), c), and d).

In one embodiment, the method further may include
f) removing the stents from the apparatus;
g) rinsing the stents;
h) immersing the stents in a passivation solution;
i) removing the stents from the passivation solution and rinsing;
j) placing the stents in a liquid and applying ultrasound energy to the liquid, or combinations thereof.

An embodiment of the electrolytic solution may include polyethylene glycol, dimethylsulfate, ethanol, and methanol.

In one embodiment, the electrolytic solution may include from about 0.1 to 5 weight percent polyethylene glycol, from about 0.5 to about 2 weight percent polyethylene glycol, or about 0.5 to I weight percent polyethylene glycol. In a further embodiment, the polyethylene glycol is PEG 1000.

In one embodiment, the electrolytic solution may include from about 0.1 to 5 weight percent polyethylene glycol, from about 0.5 to about 2 weight percent polyethylene glycol, or from about 0.5 to about 1 weight percent polyethylene glycol, wherein polyethylene glycol may be PEG 1000; from about 25 to about 60 weight percent dimethylsulfate, from about 30 to about 40 weight percent, from about 35 to about 39 weight percent, or about 37 weight percent; and from about 15 to about 50 weight percent ethanol, from about 20 to about 40 weight percent, from about 25 to about 35 weight percent, or about 28 weight percent; and a balance of methanol.

In another embodiment, in act c) the voltage is supplied for a period in the range of about 20 to about 60 seconds while the stents are immersed in the electrolytic solution.

In a further embodiment, acts b), c), and d) are repeated three to five times.

In one embodiment, the passivation solution includes nitric acid.

These and other objects and features of the present disclosure will become more fully apparent from the following description and appended claims, or may be learned by the practice of the disclosure as set forth hereinafter.

### BRIEF DESCRIPTION OF THE DRAWINGS

To further clarify the above and other advantages and features of the present disclosure, a more particular description of the disclosure will be rendered by reference to specific embodiments thereof which are illustrated in the appended drawings. It is appreciated that these drawings depict only illustrated embodiments of the disclosure and are therefore not to be considered limiting of its scope. The disclosure will be described and explained with additional specificity and detail through the use of the accompanying drawings in which:

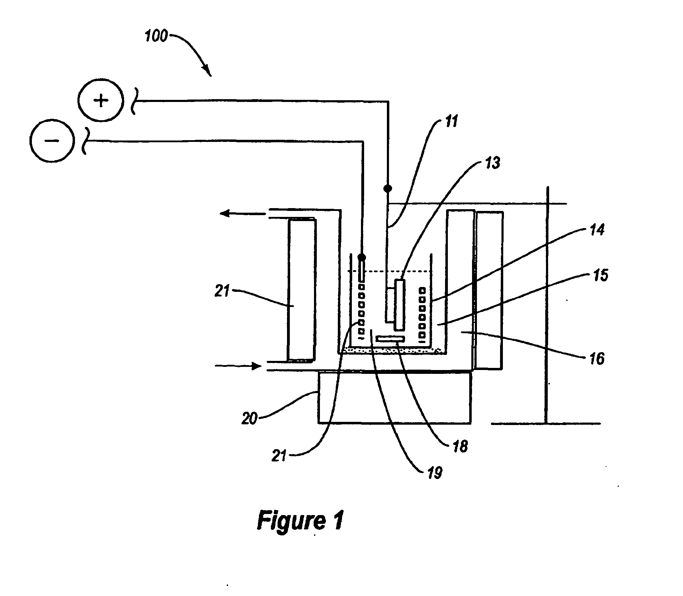

Figure 1 illustrates a view of an apparatus according to the disclosure showing the stents immersed in electrolytic solution and in close proximity to the cathode.

### DETAILED DESCRIPTION

The present disclosure is directed to an apparatus and method for electropolishing a plurality of metallic devices, in particular, metallic stents. The present disclosure may provide the advantage of simultaneously electropolishing a plurality of devices. By providing a spiral cathode in an electropolishing container made from a material with low thermal conduction, the electrolyte solutions may remain stable for an extended time.

In yet another embodiment, the apparatus includes two concentric spiral cathodes with the stents, as anodes, placed therebetween, thereby providing additional cathode surface area. The pitch of the spiral can be varied in order to balance good agitation of the electrolytic solution in the polishing container to ensure continuous electrolyte concentration and composition and provision of a high surface area of the cathode.

A novel and improved electrolytic solution for improved electropolishing of metallic medical devices, in particular stents is also described.

Referring to FIG. 1, there is shown an embodiment of an apparatus 100 according to the disclosure. The apparatus may include at least one elongated member 11 in a downward orientation along a longitudinal axis. Each of the members 11 may accommodate an electrically conductive adapter which may be capable of being removably affixed to and/or in electrical contact with a metallic stent 13.

With the at least one elongated member 11 immersed in electropolishing solution 19 contained in a container 14, a tubular continuous spiral cathode 21 may be in close proximity with the at least one elongated members 11. Each member 11 may be substantially equidistant from the facing surface of the cathode 21. This may provide a consistent field to each of the stents. Cathode 21 may be attached to a cathode collecting member. The at least one stent, as anodes, attached to elongated member(s) 11 may all be in electrical connection in series and/or in parallel with anode current conducting member. Cathode conducting member and/or anode conducting member may be connected to an electromotive force (EMF)-providing DC source, such as a battery, from which current and/or voltage may be controlled by an appropriate controller. The entire system may be placed into a polishing container 14 made from material with a low thermal conduction. Suitable materials may include glass, ceramics, plastic, or other materials. The polishing container 14 may contain the electropolishing solution 19 and/or may be placed in a double walled reaction container 16. The polishing container 14 may be cooled with a cooling solution 15 like e.g. Ethanol. Using a polishing container 14 made fmm material with a low thermal conduction water may reduce condensation at the wall portions of the polishing container exposed to air. A dilution of the polishing solution by condensing water out of the air may be reduced potentially resulting in an electropolishing solution 19 that may be stable and/or reliable and/or may provide constant polishing results. To facilitate agitation of the electropolishing solution during the electropolishing process, a stirrer 18, such as a magnetic stirrer may be placed into the polishing container 14.

Typical coronary and/or endovascular stents may vary in a range from about 7 to up to about 200 millimeters in length with a diameter in a range of about 1 to about 12 millimeters. However, stents of larger or smaller size may be suitably accommodated.

In order to accomplish the electropolishing process, the stents, which may all be identical in length, diameter, design, or combinations there of, may be placed on one or more of the adaptors. The mounted stents may be immersed into the electropolishing solution. The temperature of the electropolishing solution may be between about -20°C and about 0°C, between about -20°C and about -10°C, or about -15°C. A voltage is supplied to the stents, as anodes, and the cathode to electropolish the stents to the desired smoothness. Useful voltage may be in the range of about 7 to about 40 volts, between about 10 and about 30 volts, or between about 10 and about 20 volts. Voltage may be applied in about 20 to about 60 second intervals, in some embodiments in about 30 second intervals. However, voltages outside of these ranges may also be useful, depending upon the number of stents, electrolyte, and/or other design and/or process parameters.

It may be desirable for the electropolishing process to be performed in stages. After one immersion in the electropolishing solution, typically lasting from about 20 to about 60 seconds, the stents may be removed from the solution and washed, typically with alcohol, water, nitric acid, or combinations thereof. Then, the electropolishing may be repeated several times with each step followed by a rinse of the stents. Typically a suitable polishing process will include about three to about five iterations of the electropolishing step. But more or fewer iterations may be suitable, depending upon the stents, electrolyte, voltage, other process variations, or combinations thereof. Once the desired electropolishing is completed, the stents may be removed from the electropolishing solution and from the electropolishing apparatus, rinsed, and contacted with a passivation solution to remove residual electropolishing solution. The stents are typically again rinsed and placed in a bath to which ultrasound energy may be applied to complete the rinsing. A final rinse step may involve exposure for about 10 minutes in an ultrasound bath at approximately room temperature.

An embodiment of an electropolishing solution may include polyethylene glycol, dimethylsulfate, ethanol, and methanol.

In one embodiment, the polyethylene glycol is PEG 1000.

In a further embodiment, the electrolytic solution may include from about 0.1 to about 5 weight percent polyethylene glycol, from about 0.5 to about 2 weight percent polyethylene glycol, or about 0.5 to 1 weight percent polyethylene glycol.

In a further embodiment, the electrolytic solution includes from about 25 to about 60 weight percent dimethylsulfate, from about 30 to about 40 weight percent, from about 35 to about 39 weight percent, or from about 37 weight percent.

In one embodiment, the electrolytic solution includes from about 15 to about 50 weight percent ethanol, from about 20 to about 40 weight percent, from about 25 to about 35 weight percent, from about 25 to about 30 weight percent or about 28 weight percent; and a balance of methanol.

In one embodiment, the electrolytic solution includes from about 0.1 to about 5 weight percent polyethylene glycol, from about 0.5 to about 2 weight percent polyethylene glycol, or from about 0.5 to about 1 weight percent polyethylene glycol, the polyethylene glycol may be PEG 1000; from about 25 to about 60 weight percent dimethylsulfate, from about 30 to about 40 weight percent, from about 35 to about 39 weight percent, or about 37 weight percent; from about 15 to about 50 weight percent ethanol, from about 20 to about 40 weight percent, from about 25 to about 35 weight percent, from about 25 to about 30 weight percent, or about 28 weight percent; and a balance of methanol.

The following example is presented for the purpose of illustration and is not intended to limit the disclosure in any way.

### EXAMPLE

Four dry identical stents made from high strength medical alloy may be removably affixed to the adapters of four elongated members. While agitating the electropolishing solution (polyethylene glycol (PEG 1000) : dimethylsulfate : ethanol : methanol in a weight ratio of about 1 : 59 : 44: 54, i.e. 0.6 weight percent polyethylene glycol (PEG 1000), 37.3 weight percent dimethylsulfate, 27.9 weight percent ethanol and 34.2 weight percent methanol) the stents are lowered on the apparatus into the electropolishing solution. The positive lead from the electrical source is attached to the apparatus and the magnetic stirrer in the electropolishing container is turned on to facilitate agitation of the electropolishing solution. When the cycle time has elapsed (depending on the size and type of stent), the stents are removed from the electropolishing solution and submerged in a container of ethanol. Each stent is moved while submerged. The stents are then re-immersed in the electropolishing solution for another polishing cycle. The polishing cycle is repeated for four polishing cycles. The stents are removed from the adapters and placed into a purified water rinse for about 30 seconds. The stents are then removed and placed in nitric acid passivation rinse bath for 30 minutes. The stents are removed from the bath and placed in a purified water ultrasonic bath for about 10 minutes. The stents are then removed from the bath, rinsed with alcohol and are dried with compressed air.

The present disclosure may be embodied in other specific forms without departing from its essential characteristics. The described embodiments are to be considered in all respects only as illustrative and not restrictive. The scope of the disclosure is, therefore, indicated by the appended claims rather than by the foregoing description.

## Claims

1. A method of electropolishing metallic stents comprising:
a) affixing a stent on each of one or more of electrically conductive adaptors of an apparatus, said apparatus comprising:
at least one elongated member having a longitudinal axis, each of said at least one member comprising an electrically conductive adaptor capable of being removably affixed to and in electrical contact with a metallic stent;
an electrolytic solution;
a continuous cathode configured to be located in close proximity to each of said at least one elongated member when said a least one elongated member and cathode are immersed in said electrolytic solution;
a cathode current conducting member attached to said cathode;
an anode current conducting member wherein each of said elongated members is conductively connected electrically with said anode current conducting member;
b) immersing said at least one stent in said electrolytic solution;
c) supplying a voltage difference between said cathode current conducting member and said anode current conducting member;
d) removing said at least one stent from said solution and rinsing with alcohol; and
e) optionally, repeating acts b), c), and d);
wherein said electrolytic solution comprises polyethylene glycol, dimethylsulfate, ethanol, and methanol.

2. The method according to claim 1, further comprising f) removing said at least one stent from said apparatus;
g) rinsing said at least one stent;
h) immersing said at least one stent in a passivation solution;
i) removing said at least one stent from said passivation solution and rinsing said at least one stent; and
j) placing said at least one stent in a liquid and applying ultrasound energy to said liquid.

3. The method according to claim 1, wherein said steps b), c), and d) are repeated three to five times.

4. The method according to claim 2, wherein said passivation solution comprises nitric acid.

5. The method according to claim 2, wherein said ultrasound energy is applied to said liquid at room temperature.

6. An electrolytic solution comprising polyethylene glycol, dimethylsulfate, ethanol, and methanol.

7. The electrolytic solution according to claim 6, wherein the polyethylene glycol is PEG 1000.

8. The electrolytic solution according to claims 6 or 7, comprising 0.1 to 5 weight percent polyethylene glycol.

9. The electrolytic solution according to claim 8, comprising 0.5 to 1 weight percent polyethylene glycol.

10. The electrolytic solution according to any of claims 6 to 9, comprising 25 to 60 weight percent dimethylsulfate.

11. The electrolytic solution according to a claim 10, comprising 35 to 39 weight percent dimethylsulfate.

12. The electrolytic solution according to any of claims 6 to 11, comprising 15 to 50 weight percent ethanol and a balance of methanol.

13. The electrolytic solution according to a claim 12, comprising between 25 and 30 weight percent ethanol and a balance of methanol.

## Patentansprüche

1. Verfahren zum Elektropolieren metallischer Stents, welches das Folgende umfasst:
a) Befestigen eines Stents auf jedem von einem oder mehreren elektrisch leitfähigen Adaptern einer Vorrichtung, wobei die Vorrichtung das Folgende umfasst:
mindestens ein längliches Element, welches eine Längsachse aufweist, wobei von dem mindestens einen Element jedes einen elektrisch leitfähigen Adapter aufweist, der entfernbar an einem metallischen Stent befestigt werden und mit diesem in elektrischem Kontakt stehen kann; eine elektrolytische Lösung;
eine durchgängige Kathode, welche dafür konfiguriert ist, in enger Nachbarschaft zu jedem von dem mindestens einen länglichen Element angeordnet zu sein, wenn das mindestens eine längliche Element und die Kathode in die elektrolytische Lösung getaucht werden;
ein Kathodenstrom leitendes Element, welches an der Kathode befestigt ist;
ein Anodenstrom leitendes Element, wobei jedes der länglichen Elemente elektrisch leitend mit dem Anodenstrom leitenden Element verbunden ist;
b) Tauchen des mindestens einen Stents in die elektrolytische Lösung,
c) Zuführen einer Spannungsdifferenz zwischen dem Kathodenstrom leitenden Element und dem Anodenstrom leitenden Element;
d) Entfernen des mindestens einen Stents aus der Lösung und Spülen mit Alkohol und
e) gegebenenfalls Wiederholen der Schritte b), c) und d);
wobei die elektrolytische Lösung Polyethylenglykol, Dimethylsulfat, Ethanol und Methanol umfasst.

2. Verfahren nach Anspruch 1, welches ferner das Folgende umfasst:
f) Entfernen des mindestens einen Stents aus der Vorrichtung;
g) Spülen des mindestens einen Stents;
h) Tauchen des mindestens einen Stents in eine Passivierungslösung;
i) Entfernen des mindestens einen Stents aus der Passivierungslösung und Spülen des mindestens einen Stents und
j) Anordnen des mindestens einen Stents in einer Flüssigkeit und Anwenden von Ultraschallenergie auf die Flüssigkeit.

3. Verfahren nach Anspruch 1, wobei die Schritte b), c) und d) drei- bis fünfmal wiederholt werden.

4. Verfahren nach Anspruch 2, wobei die Passivierungslösung Salpetersäure umfasst.

5. Verfahren nach Anspruch 2, wobei die Ultraschallenergie bei Raumtemperatur auf die Flüssigkeit angewendet wird.

6. Elektrolytische Lösung, welche Polyethylenglykol, Dimethylsulfat, Ethanol und Methanol umfasst.

7. Elektrolytische Lösung nach Anspruch 6, wobei das Polyethylenglykol PEG 1000 ist.

8. Elektrolytische Lösung nach Anspruch 6 oder 7, welche 0,1 bis 5 Gewichtsprozent Polyethylenglykol umfasst.

9. Elektrolytische Lösung nach Anspruch 8, welche 0,5 bis 1 Gewichtsprozent Polyethylenglykol umfasst.

10. Elektrolytische Lösung nach einem der Ansprüche 6 bis 9, welche 25 bis 60 Gewichtsprozent Dimethylsulfat umfasst.

11. Elektrolytische Lösung nach Anspruch 10, welche 35 bis 39 Gewichtsprozent Dimethylsulfat umfasst.

12. Elektrolytische Lösung nach einem der Ansprüche 6 bis 11, welche 15 bis 50 Gewichtsprozent Ethanol und als Restmenge Methanol umfasst.

13. Elektrolytische Lösung nach Anspruch 12, welche 25 bis 30 Gewichtsprozent Ethanol und als Restmenge Methanol umfasst.

## Revendications

1. Procédé d'électropolissage de stents métalliques comprenant :
a) l'attachement d'un stent sur chacun d'un ou plusieurs adaptateurs électriquement conducteurs d'un appareil, ledit appareil comprenant :
au moins un élément allongé ayant un axe longitudinal, chacun dudit ou desdits éléments comprenant un adaptateur électriquement conducteur pouvant être attaché de manière amovible à et en contact électrique avec un stent métallique ; une solution électrolytique ;
une cathode continue configurée pour être positionnée à proximité de chacun dudit au moins un élément allongé lorsque ledit au moins un élément allongé et la cathode sont immergés dans ladite solution électrolytique ;
un élément conducteur de courant cathodique attaché à ladite cathode ;
un élément conducteur de courant anodique dans lequel chacun desdits éléments allongés est raccordé de manière conductrice et électrique audit élément conducteur de courant anodique ;
b) l'immersion dudit au moins un stent dans ladite solution électrolytique ;
c) l'alimentation d'une différence de tension entre ledit élément conducteur de courant cathodique et ledit élément conducteur de courant anodique ;
d) le retrait dudit au moins un stent de ladite solution et le rinçage avec un alcool ; et
e) la répétition facultative des étapes b), c) et d) ;
dans lequel ladite solution électrolytique comprend du polyéthylène glycol, du sulfate de diméthyle, de l'éthanol et du méthanol.

2. Procédé selon la revendication 1, comprenant en outre :
f) le retrait dudit au moins un stent dudit appareil ;
g) le rinçage dudit au moins un stent ;
h) l'immersion dudit au moins un stent dans une solution de passivation ;
i) le retrait dudit au moins un stent de ladite solution de passivation et le rinçage dudit au moins un stent ; et
j) le dépôt dudit au moins un stent dans un liquide et l'application d'une énergie ultrasonore audit liquide.

3. Procédé selon la revendication 1, dans lequel lesdites étapes b), c) et d) sont répétées trois à cinq fois.

4. Procédé selon la revendication 2, dans lequel ladite solution de passivation comprend de l'acide nitrique.

5. Procédé selon la revendication 2, dans lequel ladite énergie ultrasonore est appliquée audit liquide à température ambiante.

6. Solution électrolytique comprenant du polyéthylène glycol, du sulfate de diméthyle, de l'éthanol et du méthanol.

7. Solution électrolytique selon la revendication 6, dans laquelle le polyéthylène glycol est le PEG 1000.

8. Solution électrolytique selon la revendication 6 ou 7, comprenant de 0,1 à 5 % en poids de polyéthylène glycol.

9. Solution électrolytique selon la revendication 8, comprenant de 0,5 à 1 % en poids de polyéthylène glycol.

10. Solution électrolytique selon l'une quelconque des revendications 6 à 9, comprenant de 25 à 60 % en poids de sulfate de diméthyle.

11. Solution électrolytique selon la revendication 10, comprenant de 35 à 39 % en poids de sulfate de diméthyle.

12. Solution électrolytique selon l'une quelconque des revendications 6 à 11, comprenant de 15 à 50 % en poids d'éthanol et le reste de méthanol.

13. Solution électrolytique selon la revendication 12, comprenant de 25 à 30 % en poids d'éthanol et le reste étant du méthanol.
